# EUROPEAN PATENT SPECIFICATION

(11) **EP 1 633 330 B1**
(45) Date of publication and mention of the grant of the patent: **04.04.2007**
(21) Application number: 04770652.8
(22) Date of filing: 14.05.2004
(51) Int. Cl.: A61K 9/32

(54) **EXTENDED RELEASE OSMO-MICROSEALED FORMULATION**
OSMO-MIKROVERSCHLOSSENE ZUBEREITUNG MIT VERLÄNGERTER FREISETZUNG VON VENLAFAXINE
PREPARATION OSMO-MICROSCELLEE A LIBERATION PROLONGEE

(30) Priority: 05.06.2003 IN MU05042002
(43) Date of publication of application: 15.03.2006
(73) Proprietor: Alembic Limited, Vadodara 390003, Gujarat (IN)
(72) Inventor: BHATTACHARYA, Sampad, Alembic Limited, Vadodara 390 003, Gujarat (IN); GUMMUDAVELLI, Sridhar, Alembic Limited, Vadodara 390 003 (IN); JOSHI, Mayank, Alembic Limited, Vadodara 390 003 (IN)
(74) Representative: Newstead, Michael John
(86) International application number: PCT/IN2004/000133
(87) International publication number: WO 2004/108117

(56) References cited:
- EP-A- 0 797 991
- WO-A-01/51035
- WO-A-01/51041
- WO-A-98/47491
- US-A1- 2001 048 943
- US-A1- 2003 091 634

## Description

### Field of invention

The invention relates to extended release delivery system for pharmaceutical such as structurally novel antidepressant venlafaxine hydrochloride active as an 24 hour extended release dosage form The formulation comprises an inner solid particulate phase containing venlafaxine hydrochloride and one or more hydrophobic polymers, diluents, osmogen and binder polymers, an outer solid continues phase including one or more hydrophilic polymers and compressed into tablets and an functional coat surrounding the tablet optionally provided.

The formulation provides osmo microseal venlafaxine particles and hydrophilic matrix 24 hours extended release dosage form for better control of blood plasma level then the conventional tablet formulation which are administered two or more times a day and there are comparatively lower incidents of nausea and vomiting.

The invention also provides process of preparing osmo microseal extended release delivery system and using such system for treating human ailments such as treatment of depression.

### Background of the Invention

Venlafaxine Hydrochloride, 1-[2-(dimethylamino)-1-(4-methoxyphenyl)ethyl] cyclohexanol Hydrochloride, is an important drug in the neuro-pharmacotherapeutic arsenal used for treatment of depression. Venlafaxine and the acid addition salts thereof are disclosed in US patent 4,535,186. Venlafaxine hydrochloride is administered in compressed tablet form in doses ranging from 75 to 350 mg/day, in divided doses two or three times a day. With the plural daily dosing regimen, the most common side effect is nausea, experienced by about forty five percent of patients under treatment with Venlafaxine Hydrochloride. Vomiting also occurs in about seventeen percent of the patients. The problem is addressed in European patent 0797 991A1 and US patents 6274171, 6403120 & 6419958 which discloses an extended release once-a-daily pharmaceutical composition (American Home Products, Sherman et. al.; EFFEXOR XR^{™}) consisting of hard gelatin capsules filled with film coated spheroids comprising a therapeutically effective amount of Venlafaxine Hydrochloride, microcrystalline cellulose and, optionally, Hydroxypropyl methylcellulose extruded and spheronized and the formed spheroids further coated with a mixture of ethyl cellulose and Hydroxypropyl methylcellulose. Venlafaxine has been formulated into a controlled release dosage form with the ability to provide in a single dose a therapeutic blood serum level of the drug over a twenty four hour period. By this method, tighter plasma therapeutic range control can be obtained and a multiple dosing is avoided in this manner. The sharp peaks and troughs in blood plasma drug levels are avoided as well.

With the conventional release dosage forms of Venlafaxine Hydrochloride (tablets), peak blood plasma levels appeared after 2-4 hrs, in contrast to the extended release dosage forms, when plasma levels of Venlafaxine Hydrochloride rose after administration for between five to eight hrs (average - 6) and than begin to fall through a protracted, substantially linear decrease from the peak plasma level for the remainder of the period, maintaining therapeutic level of the drug during the entire twenty four hours period. This dosage form when tested in vitro using water at 37°C, 100 rpm and basket has the following dissolution specification,

| Time(hrs) | Mean (% drug dissolved) |
|---|---|
| 2 | <30 |
| 4 | 30-55 |
| 8 | 55-80 |
| 12 | 65-90 |
| 24 | >80 |

In fact, the art acknowledges the difficulty of producing extended release tablets by hydrogel technology because the compressed tablets were either physically unstable (poor compressibility or capping problems) or dissolved too rapidly in dissolution studies.

WO 03 / 041692 discloses an alternative approach of preparing extended release spheroids of Venlafaxine. Venlafaxine Hydrochloride is coated on a non pareil inert core, which is further coated with an inert polymer layer and subsequently with a third coat of an polymeric layer which enables the controlled release.

WO 01/51041 teaches a formulation comprising a tablet and a semi-permeable membrane surrounding the core tablet. The core comprises Venlafaxine and one osmotic agent. The semipermeable membrane surrounding the core has a passageway drilled through it either mechanically or by laser. The coated osmotic drug delivery system based tablet is further coated with an external coat comprising a therapeutically effective amount of an anti-psychotic agent.

WO 98 / 47491 teaches a novel controlled release composition and the system has been named intelliGITransporters^{™}. The composition can be formulated as an tablet or an suppository and optionally coated with an anionic polymer for enteric effect. The said coat is proposed to prevent the initial burst effect and impart the gastrointestinal tract (GIT) stealth characteristics especially in the presence of food. Prior to coating the core tablet is prepared by mixing a blend of two polymers with opposite wetting characteristics and have a water contact angle theta such that cos of theta is between +0.9848 and -0.9848. Though Venlafaxine is a part of its exhaustive list of the drugs where the proposed technology could be applicable, it does not appear in any of the example.

More recently, WO 03 / 055475 teaches a composition for once a day administration using hydrogel technology. It describes a process for the preparation of a solid controlled release pharmaceutical formulation comprising the steps of dissolving Venlafaxine and polyvinyl pyrrolidone in an aqueous solvent, applying the resulting solution onto low viscosity hydrophilic polymer, homogeneously mixing the obtained granulate with a high viscosity hydrophilic polymer, and compressing the granulate to obtain a core which is then coated with a polymeric coating comprising a water high permeable polymer and a water low permeable polymer.

### Summary of the Invention

In accordance with the present invention, a novel way has been found of formulating drug with high water solubility such as Venlafaxine Hydrochloride. Briefly, a system is used with the inner phase being an osmotic core comprising a therapeutically effective amount of Active and at least one osmotic agent, a membrane surrounding the core and the outer phase comprising of hydrophilic polymer matrix; the-blend is compressed into tablet and subsequently provided a functional coat. The combination of the inner osmo microsealed, hydrophobic core and the outer hydrophilic polymer matrix optionally with a functional coat is claimed to provide for an efficient control and modulation over the release pattern of Venlafaxine Hydrochloride.

For drug with high water solubility such as Venlafaxine Hydrochloride, one of the approach as described in European patent EP0797991 and United States patents 6274171, 6403120 and 6419958 is to formulate spheroids ofhydrophobic polymers like ethyl cellulose. Though, the process involved in the preparation of spheroids is very tedious as compared to the manufacturing of matrix tablets. In the preferred embodiment of the present invention the core is prepared by the process of granulating admixture of drug, osmogen, diluent and binder with a solution /dispersion of swellable and permeable hydrophobic polymer, and if required, the granulation is followed by coating of the granules with the said hydrophobic polymer. The coating of the granules is achieved by a process known to person of ordinary skill in the said art. The resulting granules can be sifted and resifted to remove any agglomerate produced in the coating steps. In the preferred embodiment, coating may also be achieved by repetitive re-granulation of granulated and subsequently dried mass. The formed internal phase of osmotic core is further admixed with the external phase comprising of hydrophilic polymer(s), lubricants and glidants. This system is compressed into tablets and further provided with a functional coat. The process involved in the preparation of the osmo-microsealed tablets, unlike the manufacturing of spheroids, is very simple and feasible using common equipment. Besides, inclusion of more than one rate-controlling mechanisms in one system provides for a greater control and modulation of the release pattern to achieve desired drug release profile and through it the targeted blood levels.

Some of the polymers used in the preparation of spheroids as well as the osmo-microsealed system are identical, the major difference is in the timing when the core of the present invention and the spheroid described in the prior patents are exposed to the gastrointestinal environment. Spheroids are released immediately into the system following the dissolution of the gelatin shell whereas the exposure of the osmotic core in the current invention is prolonged and regulated by the hydration of the outer hydrophilic matrix. The differential exposure of the core over a period of time provides for reduced requirement of the hydrophobic polymer level in the core and the desired level can be conveniently achieved by the process as simple as granulation. Similarly, the presence of hydrophobic polymer coating over the drug in the preparation of core provides for a reduced level of hydrophilic polymer in the external matrix of the formed tablet. Optionally, the external functional coat provides for achieving the lag phase in the drug release profile.

### Best mode for carrying the invention:

A preferred tablet composition comprises:
(i) A hydrophobic core comprised of active ingredient (Venlafaxine hydrochloride), Sodium chloride, Microcrystalline cellulose, Oleic acid, medium chain triglyceride, Povidone K 90 D and Ethyl cellulose.
(ii) A hydrophilic continuos phase consisting of Hydroxypropyl methylcellulose, Talc and Magnesium stearate.
(iii) Optionally a functional coat on the compressed tablets consisting Ammonio methacrylate copolymer, Triethyl citrate, Titanium dioxide and color.

### Brief description of drawings

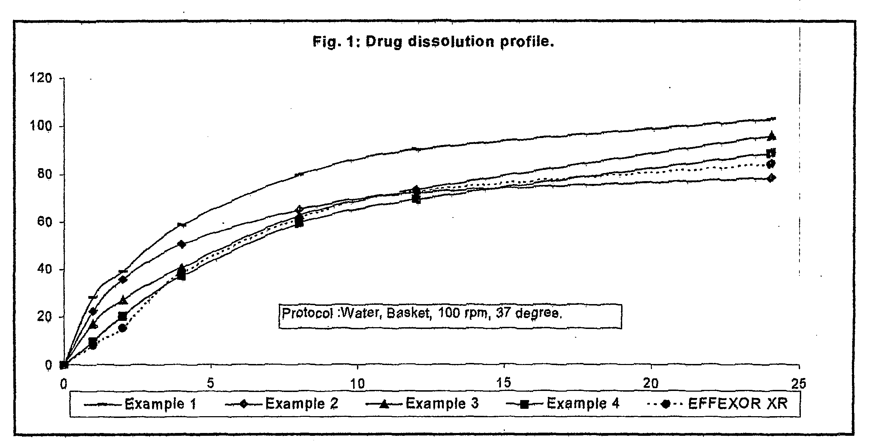

FIG. 1 is a plot showing the drug release profile of Venlafaxine Hydrochloride from four different compositions of the drug in matrices using USP I, 100 rpm and at 37°C.
FIG. 2 is a plot showing the plasma level profile of Venlafaxine Hydrochloride in Healthy Human volunteers.
FIG. 3 is a plot showing the plasma level profile of 0-desmethyl Venlafaxine Hydrochloride in HealthyHiunan volunteers.

### Detailed description of the invention

Venlafaxine hydrochloride 1 -[2-(dimethylamino)- 1 (4 methoxyphenyl)ethyl]cyclobexanol hydrochloride is polymorphic. Any of the polymorphic forms may be used in the formulations of the present invention. The invention provides for the administration of Venlafaxine in its free base, free acid, racemic, optically pure, diastereomeric and/or pharmaceutically acceptable salt forms. As used herein, "pharmaceutically acceptable salts" refer to derivatives of the disclosed compounds wherein the therapeutic compound is modified by making acid or base salts thereof. Examples of pharmaceutically acceptable salts include, but are not limited to, non-toxic mineral or organic or inorganic acid salts of venlafaxine. For example, such conventional non-toxic salts include those derived from acids such as hydrochloric, hydrobromic, sulfuric, sulfonic, sulfamic, phosphoric, nitric and the like; and the salts prepared from organic acids such as amino acids, acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The term "high water solubility" or similar term when characterizing a drug, medicament or pharmaceutical for use in the formulation of the invention refers to a solubility in water of at least about 50 mg / ml, preferably at least about 100 mg /ml or more, and more preferably greater than 150 mg / ml.

The controlled release system of the invention includes the inner solid particulate phase and the outer solid continuous phase in a weight ratio within the range from about 0.3 : 1 to about 10:1, preferably from about 0.5: 1 to about 4:1. The inner solid particulate phase contain drug in an amount within the range from about 5 % to about 75 % by weight, preferably from about 7 % to about 65 % by weight, a hydrophobic polymer in an amount within the range from about 0.5 % to about 65 % by weight, preferably from about 2 % to about 45 % by weight, an osmogen in the range from about 0.01 % to about 25 % by weight, preferably from 0.05 % to about 10 % by weight, a binder to provide strength / hardness to the particle in the range from about 0.1 % to about 10 % by weight, preferably from 0.5 % to about 8 % by weight and it may contain a pharmaceutical diluent(s) in an amount within the range from about 0 % to about 90 % by weight, preferably from 20 % to about 80 % by weight, the above percentages being based on the weight of the inner solid particulate phase.

The inner solid particulate phase have a mean particle size within the range from about 0.01 micrometer to about 2 mm, and preferably from about 50 micrometer to about 0.5 mm.

The outer continuous phase may contain one or more hydrophilic polymers in the range from about 3 % to about 60 % by weight and preferably from about 10 % to about 55 % by weight. Besides, the outer continuous phase in the various formulation of the invention may optionally include one or more fillers or excipients in an amount within the range from about 1 % to about 70 % by weight and more preferably 10 % to about 40 % by weight, the above percentages being based on the weight of the uncoated dosage form. The uncoated dosage form also contains in the outer continous phase the recommended level of glidants, lubricants, dry binders and anti-adherents.

The dosage of the invention is coated as is commonly done in the art to provide the desired functional property. The coating may comprise from about 2 to about 20 % by weight, preferably from 2.5 to 10 % by weight of the uncoated tablet core.

The hydrophobic polymer(s) insoluble in the liquids of the gastrointestinal tract, which may be employed in the inner solid particulate phase includes by way of example and without limitation, ethyl cellulose, methyl cellulose, amino methacrylate copolymer, methacrylic acid copolymers, methacrylic acid acrylic acid ethyl ester copolymer, methacrylic acid esters neutral copolymer, dimethyl aminoethyl methacrylate-methacrylic acid esters copolymer, Cellulose acetate, vinyl methyl ether/ maleic anhydride copolymers. The hydrophobic polymer is suitable for use in the form of a Non aqueous solution, aqueous suspension, an aqueous emulsion, or a water-containing organic solvent solution. They are also commercially available as, for example, Eudragit L 30D, Eudragit E30D, Aquacoat ECD-30, Surelease E-7, Eudragit RS 30D, Eudragit NE 30D, Eudragit RL 30D, etc.

Exemplary osmagens include organic and inorganic compounds such as salts, acids, bases, chelating agents, sodium chloride, lithium chloride, magnesium chloride, magnesium sulfate, lithium sulfate, potassium chloride, sodium sulfite, calcium bicarbonate, sodium sulfate, calcium sulfate, calcium lactate, d-mannitol, urea, tartaric acid, raffinose, sucrose, alpha-d-lactose monohydrate, glucose, sorbitol, combinations thereof and other similar or equivalent materials which are widely known in the art.

As used herein, the term " diluents" and "fillers" is intended to mean inert substances used as excipients to create the desired bulk, flow properties, and compression characteristics in the preparation of tablet. Such compounds include, by way of example and without limitation, dibasic calcium phosphate, kaolin, lactose, sucrose, mannitol, microcrystalline cellulose, powdered cellulose, precipitated calcium carbonate, sorbitol, and starch and other materials known to one of ordinary skill in the art.

The binder(s) used essentially to provide strength / hardness, which may be employed in the inner solid particulate phase, includes by way of example and without limitation, polyacryl amide, poly-N-vinyl amide, poly-N-vinyl-acetamide, polyvinyl pyrolidone, starch, lactose, modified corn starch, sugars, gum accacia, alginic acid, carboxymethylcellulose sodium, tragacanth, gelatin, liquid glucose, methylcellulose, pregelatinized starch, polyethylene glycol, guar gum, polysaccharide, bentonites, invert sugars, collagen, albumin, polypropylene glycol, polyoxyethylene-polypropylene copolymer, polyethylene ester, polyethylene sorbitan ester, polyethylene oxide, and hydroxypropyl methylcellulose, combinations thereof and other materials known to one of ordinary skill in the art. Important characteristics of suitable Hydroxypropyl methylcelluloses include a low viscosity, preferably less than 10 Cps and more preferably 2 to 5 Cps. Other equivalents of the Hydroxypropyl methylcelluloses 2208 and 2910 USP, having the same chemical and physical characteristics as the proprietary products named above may be substituted in the formulation.

The hydrophilic polymer(s) in the outer continuous phase includes by way of example and without limitation, hydroxyethyl cellulose, hydroxypropyl cellulose, sodium alginate, carbomer (Carbopol^{™}), sodium carboxymethyl cellulose, xanthan gum, guar gum, locust bean gum, poly vinyl acetate, polyvinyl alcohol and hydroxypropyl methylcellulose.

The functional coating layer which is optionally applied over the outer solid phase containing particles of the inner solid phase embedded therein may include one or more film-formers, such as the polymer like methacrylic acid esters neutral polymer, ethyl cellulose, cellulose acetate, polyvinyl alcohol-maleic anhydride copolymers, beta-pinene polymers, glyceryl esters of wood resins and the like. Both core tablets as well as coating formulations may contain aluminium lakes to provide color. Even the commercially available dispersion of film formers namely, Opadry, Eudragit L 30D, Eudragit E30D, Aquacoat ECD-30, Surelease E-7, Eudragit RS 30D, Eudragit NE 30D, Eudragit RL 30D, etc. may be used for the purpose of providing functional coat.

The film formers both in the inner particulate phase and on the outer continuous phase may be applied form a solvent system containing one or more solvents including water, ammonium hydroxide solution, sodium hydroxide solution, hydrochloric acid solution, alcohols like methyl alcohol, ethyl alcohol or isopropyl alcohol, ketones like acetone, or ehtylmethyl ketone, chlorinaed hydrocarbons like methylene chloride, dichloroethane, and 1,1,1-trichloroethane.

Plasticizers can also be included in the dosage form to modify the properties and characteristics of the polymers used in the coats of inner particulate phase and / or on the coat of the compressed tablet. Plasticizers useful in the invention can include, by way of example and without limitation, low molecular weight polymers, oligomers, copolymers, oils, small organic molecules, low molecular weight polyols having aliphatic hydroxyls, ester-type plasticizers, glycol ethers, poly(propylene glycol), multi-block polymers, single block polymers, low molecular weight poly(ethylene glycol), citrate ester-type plasticizers, triacetin, propylene glycol and glycerin. Such plasticizers can also include ethylene glycol, 1,2-butylene glycol, 2,3-butylene glycol, styrene glycol, diethylene glycol, triethylene glycol, tetraethylene glycol and other poly(ethylene glycol) compounds, monopropylene glycol monoisopropyl ether, propylene glycol monoethyl ether, ethylene glycol monoethyl ether, diethylene glycol monoethyl ether, sorbitol lactate, ethyl lactate, butyl lactate, ethyl glycolate, dibutylsebacate, acetyltributylcitrate, triethyl citrate, acetyl triethyl citrate, tributyl citrate and allyl glycolate. It is also contemplated and within the scope of the invention, that a combination of plasticizers may be used in the present formulation.

The dosage form of the invention can also include oils, for example, fixed oils, such as peanut oil, sesame oil, cottonseed oil, corn oil and olive oil; fatty acids, such as oleic acid, stearic acid and isostearic acid; and fatty acid esters, such as ethyl oleate, isopropyl myristate, fatty acid glycerides, medium chain triglycerides and acetylated fatty acid glycerides.

The dosage form of the invention can also comprise an antiadherent, glidant, lubricant, opaquant, colorant, polishing agents, acidifying agent, alkalizing agent, antioxidant, buffering agent and surface active agent.

Antiadherents include, by way of example and without limitation, magnesium stearate, talc, calcium stearate, glyceryl behenate, Polyethylene glycols, hydrogenated vegetable oil, mineral oil, stearic acid and other materials known to one of ordinary skill in the art.

Glidants include, by way of example and without limitation, cornstarch, talc, calcium silicate, magnesium silicate, colloidal silicon dioxide, silicon hydrogel and other materials known to one of ordinary skill in the art.

Lubricants include, by way of example and without limitation, calcium stearate, magnesium stearate, mineral oil, stearic acid, and zinc stearate and other materials known to one of ordinary skill in the art.

Opaquant may be used alone or in combination with a colorant. Such compounds include, by way of example and without limitation, titanium dioxide and other materials known to one of ordinary skill in the art.

Colorant include, by way of example and without limitation, FD&C Red No. 3, FD&C Red No. 20, FD&C Yellow No. 6, FD&C Blue No. 2, D&C Green No. 5, D&C Orange No. 5, D&C Red No. 8, caramel, and ferric oxide, red, other F.D. &, C. dyes and natural coloring agents such as grape skin extract, beet red powder, beta-carotene, annato, carmine, turmeric, paprika, and other materials known to one of ordinary skill in the art. The amount of coloring agent used will vary as desired.

Polishing agents include, by way of example and without limitation, camauba wax, and white wax and other materials known to one of ordinary skill in the art.

Acidifying agents include, by way of example and without limitation, acetic acid, amino acid, citric acid, fumaric acid and other alpha hydroxy acids, such as hydrochloric acid, ascorbic acid, and nitric acid and others known to those of ordinary skill in the art.

Alkalizing agents include, by way of example and without limitation, ammonia solution, ammonium carbonate, diethanolamine, monoethanolamine, potassium hydroxide, sodium borate, sodium carbonate, sodium bicarbonate, sodium hydroxide, triethanolamine, and trolamine and others known to those of ordinary skill in the art.

Antioxidants include, by way of example and without limitation, ascorbic acid, ascorbyl palmitate, butylated hydroxyanisole, butylated hydroxytoluene, hypophophorous acid, monothioglycerol, propyl gallate, sodium ascorbate, sodium bisulfite, sodium formaldehyde sulfoxylate and sodium metabisulfite and other materials known to one of ordinary skill in the art.

Buffering agents include, by way of example and without limitation, potassium metaphosphate, potassium phosphate, monobasic sodium acetate and sodium citrate anhydrous and dihydrate and other materials known to one of ordinary skill in the art.

The present dosage form can also employ one or more commonly known surface active agents that improve wetting of the tablet core or layers. Soaps and synthetic detergents may be employed as surfactants and as vehicles for detergent compositions. Suitable soaps include fatty acid alkali metal, ammonium, and triethanolamine salts. Suitable detergents include cationic detergents, for example, dimethyl dialkyl ammonium halides, alkyl pyridinium halides, and alkylamine acetates; anionic detergents, for example, alkyl, aryl and olefin sulfonates, alkyl, olefin, ether and monoglyceride sulfates, and sulfosuccinates; nonionic detergents, for example, fatty amine oxides, fatty acid alkanolamides, and poly(oxyethylene)-block-poly(oxypropylene) copolymers; and amphoteric detergents, for example, alkyl beta-aminopropionates and 2-alkylimidazoline quaternary ammonium salts; and mixtures thereof.

The following examples should not be considered exhaustive, but merely illustrative of only a few of the many embodiments contemplated by the present invention. The methods described herein can be followed to prepare osmo-microsealed devices according to the invention.

Examples 1 to Example 4 illustrates the development sequence to arrive at the said extended release dosage form. The composition for Example 1 to 4 is recorded in Table 1.

### Example 1

Mix Venlafaxine Hydrochloride and Microcrystalline cellulose in rapid mixer granulator for 15.0 minutes. Prepare the binder liquid by dissolving Polyvinyl Pyrolidone in the required quantity of Water with stirring Granulate the mass and mix for 10.0 minutes. Dry the above granules in a fluid bed drier and size it through a multi mill. Lubricate the sifted granules with Hydroxypropyl Methylcellulose, Talc and Magnesium stearate in a cone blender. Prepare tablets by compressing the above blend.

### Example 2

Mix Venlafaxine Hydrochloride, Microcrystalline Cellulose and Polyvinyl Pyrolidone in cone blender for 20.0 minutes. Granulate the blend with an aqueous dispersion of ethyl cellulose containing Oleic acid and medium chain triglyceride in a solution of ammonium hydroxide (Surelease E-7). Dry the granules and size it using multi mill. Lubricate the sifted granules with HydroxypropyI Methylcellulose, Talc and Magnesium stearate in a cone blender. Prepare tablets by compressing the above blend.

### Example 3

Mix Venlafaxine Hydrochloride, Microcrystalline Cellulose and Polyvinyl Pyrolidone in cone blender for 20.0 minutes. Granulate the blend with an aqueous solution of Sodium chloride in a fluid bed processor. Continue the granulation with an aqueous dispersion of ethyl cellulose containing Oleic acid and medium chain triglyceride in a solution of ammonium hydroxide (Surelease E-7). Dry the granules and size it using multi mill. Lubricate the sifted granules with Hydroxypropyl Methylcelluloswe, Talc and Magnesium stearate in a cone blender. Prepare tablets by compressing the above blend.

### Example 4

Mix Venlafaxine Hydrochloride, Microcrystalline Cellulose and Polyvinyl Pyrolidone in cone blender for 20.0 minutes. Granulate the blend with an aqueous solution of Sodium chloride in a fluid bed processor. Continue the granulation with an aqueous dispersion of ethyl cellulose containing Oleic acid and medium chain triglyceride in a solution of ammonium hydroxide (Surelease E-7). Dry the granules and size it using multi mill. Lubricate the sifted granules with Hydroxypropyl Methylcellulose, Talc and Magnesium stearate in a cone blender. Prepare tablets by compressing the above blend. Coat the tablet with an aqueous dispersion of amino methacrylate copolymer containing Triethyl citrate, Talc and Titanium dioxide.

The composition for Example 5 to 12 is recorded in Table 2, which illustrates the various combinations, and the processes, which can be used to prepare the claimed dosage form.

### Example 5

Mix Venlafaxine Hydrochloride, Copolyvidone, Lactose and Mannitol in RMG for 15.0 minutes. Prepare the film forming liquid by dissolving Cellulose acetate and polyethylene glycol into the required quantity of Dichloromethane: Isopropyl alcohol (2:1) with stirring. Granulate the mass with partial quantity of the film forming liquid and mix for 30.0 minutes. Dry the above granules in a fluid bed drier. Re-granulate the mass with the remaining quantity of the film forming liquid. Repeat the process and dry the granules to achieve the desired film coating of the granules. Size the granules using multi mill. Lubricate the sifted granules with Carbomer, Dibasic Calcium Phosphate and Glyceryl behenate in a cone blender. Compress the above blend into tablets and coat them with a freshly prepared aqueous dispersion of Eudragit RS, Triethyl citrate, Talc and Titanium dioxide in water.

### Example 6

Mix Venlafaxine Hydrochloride, Lactose and Mannitol in cone blender. Prepare the film forming liquid by dispersing Cellulose acetate and polyethylene glycol into the required quantity of Dichloromethane: Isopropyl alcohol (2:1) with stirring Granulate the blended mass with an aqueous solution of Copolyvidone in a fluid bed processor. Continue the granulation with the film forming solution in a fluid bed processor. Size the granules using multi mill. Lubricate the granules with Carbomer, Dibasic Calcium Phosphate and Glyceryl behenate in a cone blender. Compress the above blend into tablets and coat them with a freshly prepared dispersion of Eudragit RS, Triethyl citrate, Talc and, Titanium dioxide in water.

### Example 7

Mix Venlafaxine Hydrochloride, Lactose and Mannitol in cone blender for 8.0 minutes. Granulate the mass with an aqueous solution of Hydroxypropyl Methylcellulose (Methocel E3) in a fluid bed processor. Prepare the film forming liquid by dissolving Cellulose acetate and polyethylene glycol into the required quantity of Dichloromethane: Isopropyl alcohol (2:1) with stirring. Coat the dried granules with the film forming solution in a Wurster fluid bed processor. Size the granules using multi milL Lubricate the coated granules with Hydroxypropyl Methylcellulose, Dibasic Calcium Phosphate, Magnesium Stearate and Talc in a cone blender. Compress the above blend into tablets and coat them with a freshly prepared dispersion of Eudragit RS, Triethyl citrate, Talc and Titanium dioxide in water.

### Example 8

Mix Venlafaxine Hydrochloride, Microcrystalline Cellulose and Lactose in RMG for 20.0 minutes. Granulate the mass with an aqueous solution of Sodium chloride. Dry the granules in a fluid bed drier. Coat the dried granules in a Wurster fluid bed processor with the aqueous dispersion of ethyl cellulose containing Oleic acid and medium chain triglyceride in aqueous solution of ammonium hydroxide (Surelease E 7). Size the granules using multi mill. Lubricate the sifted granules with Hydroxypropyl Methylcellulose, Carbomer 934 P, Magnesium Stearate and Talc in a cone blender. Compress the above blend into tablets and coat them with a freshly prepared dispersion of Eudragit RL, Triethyl citrate, Talc and Titanium dioxide in water.

### Example 9

Mix Venlafaxine Hydrochloride and Mannitol in RMG for 5.0 minutes. Granulate the mass with an aqueous solution of Povidone. Dry the granules in a fluid bed drier. Prepare the film forming liquid by dispersing Cellulose acetate and polyethylene glycol into the required quantity of Dichloromethane: Isopropyl alcohol (2:1) with stirring. Coat the dried granules with the film forming solution in a Wurster fluid bed processor. Lubricate the coated granules with Hydroxypropyl Methylcellulose, Dibasic Calcium Phosphate, Magnesium stearate and Talc in a cone blender. Compress the above blend into tablets and coat them with a freshly prepared dispersion of Eudragit RL, Eudragit RS, Triethyl citrate, Talc and Titanium dioxide in water.

### Example 10

Mix Venlafaxine Hydrochloride, sodium chloride and Microcrystalline Cellulose in RMG for 5.0 minutes. Granulate the mass with an aqueous solution of Povidone. Dry the granules in a fluid bed drier. Prepare the film forming liquid by dispersing Cellulose acetate and polyethylene glycol into the required quantity of Dichloromethane: Isopropyl alcohol (2:1) with stirring. Coat the dried granules with the film forming solution in a Wurster fluid bed processor. Lubricate the coated granules with Hydroxypropyl Methylcellulose, Dibasic Calcium Phosphate and glyceryl behenate in a cone blender. Compress the above blend into tablets and coat them with a freshly prepared dispersion of Eudragit RL, Eudragit RS, Triethyl citrate, Talc and Titanium dioxide in water.

### BIOAVAILABILITY STUDIES

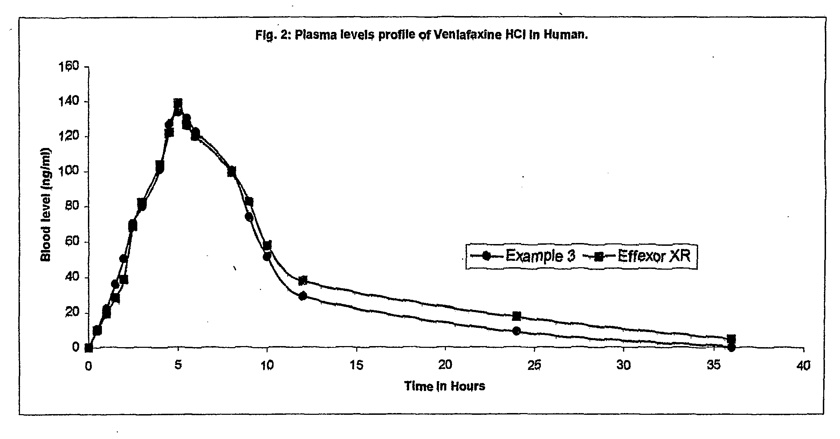

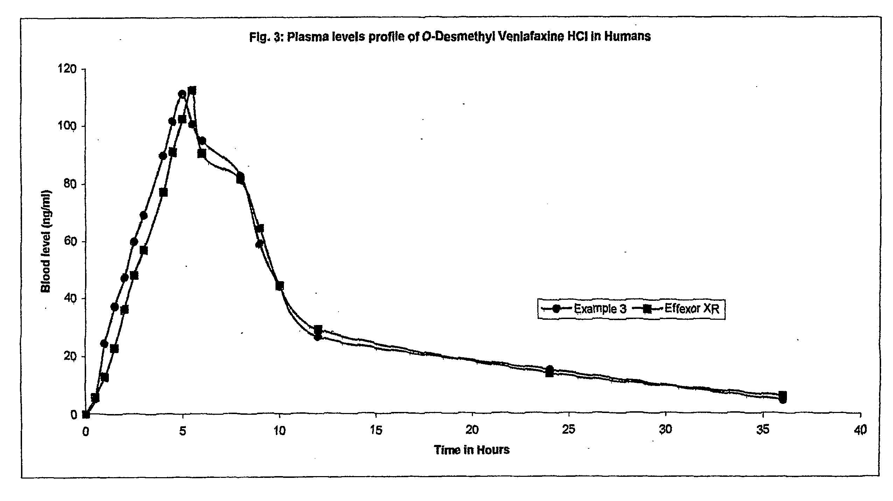

A randomized, two-treatment, two-period, two-sequence, single dose, crossover bioavailability study on Venlafaxine 150 mg extended release tablets (Example 3), compared with Venlafaxine 150 mg extended release capsule (Effexor XR^{™}) manufactured by Wyeth-Ayerst, in six, healthy, adult, male, human subjects was conducted under non fasting conditions. The extended release plasma level profile of Venlafaxine and its Active metabolite O-desmethyl Venlafaxine is demonstrated in Fig. 2 and Fig. 3 respectively.

**Table 1**

| **Sr. No.** | **EXAMPLE** | **Percentage w/w** | | | |
|---|---|---|---|---|---|
| | | **1** | **2** | **3** | **4** |
| 1 | Venlafaxine Hydrochloride | 21.38 | 21.38 | 21.38 | 19.44 |
| 2 | Microcrystalline cellulose | 17.75 | 17.75 | 16.25 | 14.77 |
| 3 | Polyvinyl pyrolidone | 1.66 | 1.66 | 1.66 | 1.51 |
| 4 | Sodium Chloride | -- | -- | 1.50 | 1.36 |
| 5 | Medium Chain Triglycerides | -- | 0.86 | 0.86 | 0.78 |
| 6 | Ethyl Cellulose | -- | 13.85 | 13.85 | 12.59 |
| 7 | Oleic Acid | -- | 1.75 | 1.75 | 1.59 |
| 8 | Ammonium Hydroxide (28%) | -- | Lost in Processing | Lost in Processing | Lost in Processing |
| 9 | Purified Water | Lost in Processing | Lost in Processing | Lost in Processing | Lost in Processing |
| 10 | Hydroxypropyl Methylcellulose | 57.71 | 41.25 | 41.25 | 37.50. |
| 11 | Magnesium Stearate | 1 | 1 | 1 | 0.91 |
| 12 | Talc | 0.5 | 0.5 | 0.5 | 1.98 |
| 13 | Trimethyl amino methacrylate copolymer, Type A | -- | -- | -- | 5.35 |
| 14 | Triethyl citrate | -- | -- | -- | 1.07 |
| 15 | Titanium dioxide | -- | -- | -- | 1.14 |

**Table 2**

| **Sr. No.** | **EXAMPLE** | **Percentage w/w** | | | | |
|---|---|---|---|---|---|---|
| | | **5-6** | **7** | **8** | **9** | **10** |
| 1 | Venlafaxine Hydrochloride | 39.06 | 38.46 | 20.46 | 21.25 | 22.70 |
| 2 | Microcrystalline cellulose | -- | -- | 8.00 | -- | 13.3 |
| 3 | Lactose | 17.47 | 17.07 | 10.49 | -- | -- |
| 4 | Polyvinyl pyrolidone | -- | -- | -- | 3.13 | 2.67 |
| 5 | Copolyvidone | 2.44 | -- | -- | -- | -- |
| 6 | Hydroxypropyl methylcellulose | -- | 1.22 | -- | -- | -- |
| 7 | Sodium Chloride | -- | -- | 0.66 | -- | 4.00 |
| 8 | Mannitol | 3.66 | 1.22 | -- | 1.25 | -- |
| 9 | Medium Chain Triglycerides | -- | -- | 0.24 | -- | -- |
| 10 | Ethyl Cellulose | -- | -- | 5.88 | -- | -- |
| 11 | Cellulose acetate | 8.38 | 5.25 | -- | 17.67 | 8.63 |
| 12 | Oleic Acid | -- | -- | 0.49 | -- | -- |
| 13 | Polyethylene glycol | 1.46 | 0.73. | -- | 0.25 | 1.1 |
| 14 | Aminonium Hydroxide (28%) | -- | -- | Lost in Processing | -- | -- |
| 15 | Purified Water / Isopropyl alcohol/Dichloro methane | Lost in Processing | Lost in Processing | -- | Lost in Processing | Lost in Processing |
| 16 | Hydroxypropyl Methylcellulose | -- | 17.07 | 29.63 | 30.88 | -- |
| 17 | Carbomer | 12.20 | -- | 4.32 | -- | 23.63 |
| 18 | Dibasic Calcium Phosphate | 10.73 | 11.46 | -- | 12.50 | 16.90 |
| 19 | Magnesium Stearate | - | 1.46 | 1.95 | 0.63 | -- |
| 20 | Glyceryl behenate | 1.22 | -- | -- | -- | 2.00 |
| 21 | Talc | 0.55 | 1.82 | 3.75 | 2.55 | 0.83 |
| 22 | Trimethyl amino methacrylate copolymer, Eudragit RL | -- | -- | 10.0 | 3.00 | 2.00 |
| 23 | Triméthyl amino methacrylate copolymer, Eudragit RS | 2.00 | 3.00 | -- | 4.00 | 1.00 |
| 24 | Triethyl citrate | 0.40 | 0.60 | 2.00 | 1.40 | 0.60 |
| 25 | Titanium dioxide | 0.43 | 0.64 | 2.13 | 1.49 | 0.64 |

## Claims

1. An extended release osmo microsealed formulation comprising of an inner solid osmo-microsealed particulate phase consisting of a therapeutically effective amount of venlafaxine Active or salt thereof and at least one osmogen/osmotic agent or osmo polymer, a diluent, a binder and a hydrophobic polymer membrane forming the core; an outer solid continous phase consisting of hydrophilic water soluble and /or swellable polymer, compressed into tablets and optionally coated with a functional coat.

2. The formulation of claim 1, wherein the inner osmo microsealed particulate phase and the outer continuous phase is in a ratio within the range of 0.3 : 1 to 10 : 1, preferably from 0.5 : 1 to about 4 : 1.

3. The formulation of claim 1, wherein the inner solid particulate phase contain active drug or salt there of in an amount within the range from about 5% to 75%, preferably from about 7 % to 65 % by weight, ethyl cellulose and / or cellulose acetate in an amount within the range from 0.5 % to 65 % by weight, preferably from 2 % to 45 % by weight sodium chloride and / or mannitol in the range from 0.01 % to 25. % by weight, preferably from 0.05 % to 10 % by weight, Polyvinyl pyrolidone and / or Hydroxypropyl methyl cellulose (low viscosity) in the range from 0.1 % to 10 % by weight, preferably from 0.5 % to 8 % by weight and it may contain microcrystalline cellulose and / or lactose in an amount within the range from about 0 % to 90 % by weight, preferably from 20 % to 80 % by weight, the above percentages being based on the weight of the inner solid particulate phase.

4. The formulation of claim 1, wherein the inner solid particulate phase contains the hydrophobic polymer in an amount within the range from about 0.5% to 65% by wt. preferable from about 2% to 45% by wt. of the inner solid particulate phase.

5. The formulation of claim 4, wherein the hydrophobic polymer is used in the form of a non-aqueous solution, aqueous suspension, an aqueous emulsion or a water containing organic solvent solution.

6. The formulation of claim 4, wherein the hydrophobic polymer is selected from ethyl cellulose, methyl cellulose, amino methacrylate copolymer, methacrylic acid copolymers, methacrylic acid acrylic acid ethyl ester copolymer, methacrylic acid esters neutral copolymer, dimethyl aminoethyl methacrylate-methacrylic acid esters copolymer, Cellulose acetate, vinyl methyl ether/maleic anhydride copolymers.

7. The formulation of claim 1, wherein the inner solid particulate phase contains osmogen in an amount within the range from about 0.01 % to about 25% by wt.. preferably from 0.05% to about 10% by wt of the inner solid particulate phase.

8. The formulation of claim 7, wherein the osmogens include organic and inorganic compounds such as salts, acids, bases, chelating agents, sodium chloride, lithium chloride, magnesium chloride, magnesium sulfate, lithium sulfate, potassium chloride, sodium sulfite, calcium bicarbonate, sodium sulfate, calcium sulfate, calcium lactate, d-mannitol, urea, tartaric acid, raffinose, sucrose, alpha-d-lactose monohydrate, glucose, sorbitol and the other similar or equivalent materials and combination thereof.

9. The formulation of claim 1, wherein the inner solid particulate phase contains a binder in the range from about 0.1 % to about 10% by wt. preferably from 0.5% to about 8% by wt of the inner solid particulate phase.

10. The formulation of claim 9, wherein the binder is selected from polyacryl amide, poly-N-vinyl amide, poly-N-vinyl-acetamide, polyvinyl pyrolidone, starch, lactose, modified com starch, sugars, gum accacia, alginic acid, carboxymethylcellulose sodium, tragacanth, gelatin, liquid glucose, methylcellulose, pregelatinized starch, polyethylene glycol, guar gum, polysaccharide, bentonites, invert sugars, collagen, albumin, polypropylene glycol, polyoxyethylen-polypropylene copolymer, polyethylene ester, polyethylene sorbitan ester, polyethylene oxide, and hydroxypropyl methylcellulose and the other similar or equivalent materials or combination thereof.

11. The formulation of claim 10, wherein the viscosity of hydroxypropyl methylcellulose are of low viscosity preferably less than 0,01 Pa.s (10 Cps) and more preferably 0,002 to 0,005 Pa.s (2 to 5 Cps).

12. The formulation of claim 1, wherein the inner solid particulate phase contains diluent in an amount within the range from about 0 to 90% by wt or preferably from about 20% to 80% by wt of the inner solid particulate phase.

13. The formulation of claim-12, wherein the diluent is an inert substance used as excipients to create the desired bulk flow properties and compression. characteristic required in the preparation of tablets.

14. The formulation of claim 12, wherein the diluent is selected from dibasic calcium phosphate, kaolin, lactose, sucrose, mannitol, microcrystalline cellulose, powdered cellulose, precipitated calcium carbonate, sorbitol, and starch and the like materials.

15. The formulation of claim 1, wherein the inner solid particulate phase has a mean particle size within the range from about 0.01 micrometer to about 2mm, and preferably from about 50 micrometer to about 0.5 mm.

16. The formulation of claim 1, wherein the said outer solid continous phase contains hydrophilic polymers in an amount within the range from about 3% to 60% by wt and preferably from about 10% to 55% by wt of the uncoated dosage form/tablet.

17. The formulation of claim 16, wherein the hydrophilic polymer is selected from hydroxyethyl cellulose, hydroxypropyl cellulose, sodium alginate, carbomer (Carbopol^{™}), sodium carboxymethyl cellulose, xanthan gum, guar gum, locust bean gum, poly vinyl acetate, polyvinyl alcohol and hydroxypropyl methylcellulose.

18. The formulation of claim 16, wherein the said outer solid continous phase include one or more fillers or excipients in an amount within the range from about 1% to 70% by wt. and more preferably 10% to 40% by wt of the uncoated dosage form/tablet.

19. The formulation of claim 16, wherein the said outer sold continous phase includes the recommended level of glidants, lubricants, dry binders , anti adherents.

20. The formulation of claim 1, wherein the functional coat provided optionally is about 2% to 20% by wt preferably from 2.5% to 10% by wt. of the uncoated tablet core.

21. The formulation of claim 20, wherein the functional coating layer optionally provided over the outer solid continous phase containing particulates of inner-solid phase embedded therein, include one ore more film formers such as methacrylic acid esters neutral polymer, ethyl cellulose, cellulose acetate, polyvinyl alcohol-maleic anhydride copolymers, beta-pinene polymers, glyceryl esters of wood resins and the like.

22. The formulation of claim 20, wherein suitable colouring agent are added in the coating.

23. The formulation of claim 1, wherein plastizers are included to modify the properties and chracteristic of polymers used in the coats of inner particulate phase and/or on the coat of compressed tablets.

24. The formulation of claim 23, wherein the plastizers are selected from low molecular wt polymers, low molecular weight polymers, oligomers; copolymers, oils, small organic molecules, low molecular weight polyols having aliphatic hydroxyls, ester-type plasticizers, glycol ethers, poly(propylene glycol), multi-block polymers, single block polymers, low molecular weight poly(ethylene glycol), citrate ester-type plasticizers, triacetin, propylene glycol and glycerin, where such plasticizers can also include ethylene glycol, 1,2-butylene glycol, 2,3-butylene glycol, styrene glycol, diethylene glycol, triethylene glycol, tetraethylene glycol and other poly(ethylene glycol) compounds, monopropylene glycol monoisopropyl ether, propylene glycol-monoethyl ether, ethylene glycol monoethyl ether, diethylene glycol monoethyl ether, sorbitol lactate, ethyl lactate, butyl lactate, ethyl glycolate, dibutylsebacate, acetyltributylcitrate, triethyl citrate, acetyl triethyl citrate, tributyl citrate and allyl glycolate or combination thereof.

25. The formulation of claim 24, wherein oils used are selected from peanut oil, sesame oil, cottonseed oil, com oil and olive oil; fatty acids, such as oleic acid, stearic acid and isostearic acid; and fatty acid esters, such as ethyl oleate, isopropyl myristate, fatty acid glycerides, medium chain triglycerides and acetylated fatty acid glycerides.

26. The formulation of claim 1, wherein the dosage form/tablet includes antiadherent, glidant, lubricant, opaquant, colorant, polishing agents, acidifying agent, alkalizing agent, antioxidant, buffering agent and surface active agent.

27. The formulation of claim 26, wherein the antiadherent are selected from magnesium stearate, talc, calcium stearate, glyceryl behenate, Polyethylene glycols, hydrogenated vegetable oil, mineral oil, stearic acid and the like materials. .

28. The formulation of claim 26, wherein the glidant are selected from cornstarch, talc, calcium silicate, magnesium silicate, colloidal silicon dioxide, silicon hydrogel and the like materials.

29. The formulation of claim 26, wherein the lubricant are selected from calcium stearate, magnesium stearate, mineral oil, stearic acid, and zinc stearate and the like materials.

30. The formulation of claim 26, wherein opaquant is used alone or in combination with colorant such as Titaniurridioxide and the like materials.

31. the formulation of claim 26, wherein the colorant are selected from FD&C Red No. 3, FD&C Red No. 20, FD&C Yellow No. 6, FD&C Blue No. 2, D&C Green No. 5, D&C Orange No. 5, D&C Red No. 8, caramel, and ferric oxide, red, other F.D. & C. dyes and natural coloring agents such as grape skin extract, beet red powder, beta-carotene, annato, carmine, turmeric, paprika and the like materials.

32. The formulation of claim 26, wherein the polising agent are selected from camauba wax, white wax and the like materials.

33. the formulation of claim 26, wherein the acidifying agent are selected from acetic acid, amino acid, citric acid, fumaric acid and other alpha hydroxy acids, such as hydrochloric acid, ascorbic acid, and nitric acid and the like materials.

34. The formulation of claim 26, wherein the alkalizing agent are selected from ammonia solution, ammonium carbonate, diethanolamine, monoethanolamine, potassium hydroxide, sodium borate, sodium carbonate, sodium bicarbonate, sodium hydroxide, triethanolamine, and trolamine and the like materials.

35. The formulation of claim 26, wherein the antioxidants are selected from ascorbic acid, ascorbyl palmitate, butylated hydroxyanisole, butylated, hydroxytoluene, hypophophorous acid, monothioglycerol, propyl gallate, sodium ascorbate, sodium bisulfite, sodium formaldehyde sulfoxylate and sodium metabisulfite and the like materials.

36. The formulation of claim 26, wherein the buffering agent are selected from potassium metaphosphate, potassium phosphate, monobasic sodium acetate and sodium citrate anhydrous and dehydrate and the like materials.

37. The formulation of claim 1, wherein the dosage form/tablet includes surfaces active agent that improve wetting of the tablet core or coating layers.

38. The formulation of claim 37, wherein the surface active agent are soaps and synthetic detergents.

39. The formulaation of claim 38, wherein the soaps include fatty acid alkali metal, ammonium, and triethanolamine salts.

40. The formulation of claim 38, wherein the detergents are cationic detergents, for example, dimethyl dialkyl ammonium halides, alkyl pyridinium halides, and alkylamine acetates; anionic detergents, for example, alkyl, aryl and olefin sulfonates, alkyl, olefin, ether and monoglyceride sulfates, and sulfosuccinates; nonionic detergents, for example, fatty amine oxides, fatty acid alkanolamides, and poly(oxyethylene)-block-poly(oxypropylene) copolymers; and amphoteric detergents, for example, alkyl .beta-aminopropionates and 2-allrylimidazoline quaternary ammonium salts; and mixtures thereof.

41. A process of preparing an extended release osmo-microsealed formulation , comprising the following steps:
i. forming osmo micrsealed inner solid particulate phase by granulation of venlafaxin active or salt thereof with one or more diluents to increase the bulk, binder to provide strength/hardness to the particulate one or more osmogen for generating osmatic pressure across the hydrophobic coating and hydrophobic polymer.
ii. embedding the inner solid particulate phase in an outer solid continous phase including one or more hydrophilic polymers.
iii. compressing the biphasic blend into tablet.
iv. coating the tablet optionally with a functional coat containing polymers.

42. A process as claimed in claim 41, wherein the inner osmo microsealed particle coat is obtained by granulation of drug, diluent, binder and osmogen mixture with the dispersion of the coating polymer forming a matrix of drug, diluent, osmogen and the polymer. If required, the granules are re-granulated till the entire coat is applied.

43. A process as claimed in claim 41, wherein the inner osmo microsealed particle coat is obtained by granulation of drug, diluent and binder with the solution of osmogen. The granulation is further continued with the dispersion of hydrophobic ,polymer. If required, the granules are re-granulated till the entire coat is applied.

44. A process as claimed in claim 41, wherein the inner osmo microsealed particle coat is obtained by granulation of drug, diluent and osmogen with the solution of binder. The granulation is further continued with the dispersion of hydrophobic polymer. If required, the granules are re-granulated till the entire coat is applied.

45. A process as claimed in claim 41, wherein the inner osmo microsealed particle coat is obtained by partial granulation of the drug, diluent and osmogen mixture with the dispersion of coating polymer forming a matrix of drug, diluent, osmogen and the polymer. The granules are further coated on a fluid bed processor with the remaining quantity of the hydrophobic polymer.

46. A process as claimed in claim 41, wherein the inner osmo microsealed particle are obtained by granulation of the drug, osmogen and binder. The granules are further coated on a fluid bed processor with the hydrophobic coating polymer.

47. A process as claimed in claim 41, wherein the inner osmo microsealed particle are obtained by granulation of the drug, binder and diluent using a solution of osmogen. The granules are further coated on a fluid bed processor with the hydrophobic coating polymer

48. A process as claimed in claim 41, wherein the inner osmo microsealed particle are obtained by extrusion-spheronixation of wet blended mass of drug, binder, diluent and osmogen. The mini spherules obtained are further coated on a fluid bed processor with the hydrophobic coating polymer.

## Patentansprüche

1. Osmo-mikroversiegelte Rezeptur verlängerter Freisetzung umfassend eine innere feste osmo-mikroversiegelte teilchenförmige Phase bestehend aus einer therapeutisch wirksamen Menge Venlafaxin aktiv oder eines Salzes desselben und mindestens ein osmogenes /osmotisches Mittel oder Osmopolymer, ein Verdünnungsmittel, ein Bindemittel und eine hydrophobe Polymermembran, die den Kern bildet; eine äußere feste kontinuierliche Phase bestehend aus hydrophilem, wasserlöslichem und/oder quellbarem Polymer, das zu Tabletten komprimiert und wahlweise mit einer funktionellen Beschichtung beschichtet ist.

2. Rezeptur nach Anspruch 1, wobei die innere osmo-mikroversiegelte teilchenförmige Phase und die äußere kontinuierliche Phase in einem Verhältnis im Bereich von 0,3 : 1 bis 10 : 1, bevorzugt 0,5 : 1 bis etwa 4 : 1 stehen.

3. Rezeptur nach Anspruch 1, wobei die innere feste teilchenförmige Phase Folgendes enthält:
ein aktives Arzneimittel oder ein aktives Salz desselben in einer Menge innerhalb des Bereichs von etwa 5 Gew.-% bis 75 Gew.-%, bevorzugt etwa 7 Gew.-% bis 65 Gew.-%,
Ethylcellulose und/oder Celluloseacetat in einer Menge innerhalb des Bereichs von 0,5 Gew.-% bis 65 Gew.-%, bevorzugt 2 Gew.-% bis 45 Gew.-% Natriumchlorid und/oder Mannit im Bereich von 0,01 Gew.-% bis 25 Gew.-%, bevorzugt 0,05 Gew.-% bis 10 Gew.-%, Polyvinylpyrrolidon und/oder Hydroxypropylmethylcellulose (geringer Viskosität) im Bereich von 0,1 Gew.-% bis 10 Gew.-%, bevorzugt 0,5 Gew.-% bis 8 Gew.-% und sie mikrokristalline Cellulose und/oder Lactose in einer Menge innerhalb des Bereichs von etwa 0 Gew.-% bis 90 Gew.-%, bevorzugt 20 Gew.-% bis 80 Gew.-% enthalten kann, wobei die obigen Prozentsätze auf das Gewicht der inneren festen teilchenförmigen Phase bezogen sind.

4. Rezeptur nach Anspruch 1, wobei die innere feste teilchenförmige Phase hydrophobes Polymer in einer Menge innerhalb des Bereichs von etwa 0,5 Gew.-% bis 65 Gew.-%, bevorzugt etwa 2 Gew.-% bis 45 Gew.-%, auf die innere feste teilchenförmige Phase bezogen, enthält.

5. Rezeptur nach Anspruch 4, wobei das hydrophobe Polymer in Form einer nichtwässrigen Lösung, wässrigen Suspension, einer wässrigen Emulsion oder einer wasserhaltigen organischen Lösungsmittellösung verwendet wird.

6. Rezeptur nach Anspruch 4, wobei das hydrophobe Polymer unter Ethylcellulose, Methylcellulose, Aminomethacrylatcopolymer, Methacrylsäurecopolymeren, Methacrylsäure-Acrylsäure-Ethylestercopolymer, neutralem Methacrylsäureester-Copolymer, Dimethylaminoethyl-Methacrylat-Methacrylsäureester-Copolymer, Celluloseacetat, Vinylmethylether-Maleinsäure-Copolymer ausgewählt wird.

7. Rezeptur nach Anspruch 1, wobei die innere feste teilchenförmige Phase Osmogen in einer Menge innerhalb des Bereichs von etwa 0,01 Gew.-% bis etwa 25 Gew.-%, bevorzugt 0,05 Gew.-% bis etwa 10 Gew.-% , auf die innere feste teilchenförmige Phase bezogen, enthält.

8. Rezeptur nach Anspruch 7, wobei die Osmogene organische und anorganische Verbindungen wie Salze, Säuren, Basen, Chelatbildner, Natriumchlorid, Lithiumchlorid, Magnesiumchlorid, Magnesiumsulfat, Lithiumsulfat, Kaliumsulfat, Natriumsulfit, Calciumcarbonat, Natriumsulfat, Calciumsulfat, Calciumlactat, d-Mannit, Harnstoff, Weinsäure, Raffinose, Saccharose, Alpha-d-lactosemonohydrat, Glucose, Sorbit und andere ähnliche oder äquivalente Materialien und Kombinationen derselben umfassen.

9. Rezeptur nach Anspruch 1, wobei die innere feste teilchenförmige Phase ein Bindemittel im Bereich von etwa 0,1 Gew.-% bis etwa 10 Gew.-%, bevorzugt 0,5 Gew.-% bis etwa 8 Gew.-%, auf die innere feste teilchenförmige Phase bezogen, enthält.

10. Rezeptur nach Anspruch 9, wobei das Bindemittel unter Polyacrylamid, Poly-N-vinylamid, Poly-N-vinylacetamid, Polyvinylpyrrolidon, Stärke, Lactose, modifizierter Maisstärke, Zuckern, Gummi arabicum, Algininsäure, Carboxymethylcellulose-Natrium, Tragant, Gelatine, flüssiger Glukose, Methylcellulose, vorgelatinierter Stärke, Polyethylenglykol, Guargummi, Polysaccharid, Bentoniten, Invertzuckern, Collagen, Albumin, Polypropylenglykol, Polyoxyethylen-Polypropylen-Copolymer, Polyethylenester, Polyethylensorbitanester, Polyethylenoxid und Hydroxypropylmethylcellulose und anderen ähnlichen oder äquivalenten Materialien oder Kombinationen derselben ausgewählt wird.

11. Rezeptur nach Anspruch 10, wobei die Viskosität von Hydroxypropylmethylcellulose eine geringe Viskosität, bevorzugt von weniger als 0,01 Pa.s (10 cP) und noch bevorzugter 0,002 bis 0,005 Pa.s (2 bis 5 cP) ist.

12. Rezeptur nach Anspruch 1, wobei die innere feste teilchenförmige Phase Verdünnungsmittel in einer Menge innerhalb des Bereichs von 0 bis 90 Gew.-% oder bevorzugt etwa 20 Gew.-% bis 80 Gew.-%, auf die innere feste teilchenförmige Phase bezogen, enthält.

13. Rezeptur nach Anspruch 12, wobei das Verdünnungsmittel eine inerte Substanz ist, die als Vehikel zum Bilden der erwünschten Massenflusseigenschaften und Komprimiercharakteristik verwendet wird, die bei der Herstellung von Tabletten erforderlich sind.

14. Rezeptur nach Anspruch 12, wobei das Verdünnungsmittel unter zweibasigem Calciumphosphat, Kaolin, Lactose, Saccharose, Mannit, mikrokristalliner Cellulose, pulverförmiger Cellolose, ausgefälltem Calciumcarbonat, Sorbit und Starke und derartigen Materialien ausgewählt wird.

15. Rezeptur nach Anspruch 1, wobei die innere feste teilchenförmige Phase eine durchschnittliche Teilchengröße innerhalb des Bereichs von etwa 0,01 Mikrometern bis etwa 2 mm, und bevorzugt etwa 50 Mikrometern bis etwa 0,5 mm aufweist.

16. Rezeptur nach Anspruch 1, wobei die äußere feste kontinuierliche Phase hydrophile Polymere in einer Menge innerhalb des Bereichs von etwa 3 Gew.-% bis 60 Gew.-% und bevorzugt etwa 10 Gew.-% bis 55 Gew.-%, auf die unbeschichtete Dosierform/Tablette bezogen, enthält.

17. Rezeptur nach Anspruch 16, wobei das hydrophile Polymer unter Hydroxyethylcellulose, Hydroxypropylcellulose, Natriumalginat, Carbomer (Carbopol^{wz}), Natriumcarboxymethylcellulose, Xanthangummi, Guargummi, Johannisbrotkernmehl, Polyvinylacetat, Polyvinylalkohol und Hydroxypropylmethylcellulose ausgewählt wird.

18. Rezeptur nach Anspruch 16, wobei die äußere feste kontinuierliche Phase einen oder mehrere Füllstoffe oder Vehikel in einer Menge innerhalb des Bereichs von etwa 1 Gew.-% bis 70 Gew.-% und noch bevorzugter 10 Gew.-% bis 40 Gew.-%, auf die unbeschichtete Dosierform/Tablette bezogen, umfasst.

19. Rezeptur nach Anspruch 16, wobei die äußere feste kontinuierliche Phase das empfohlene Niveau an Gleitmitteln, Schmiermitteln, trockenen Bindemitteln, Antiadhärenten umfasst.

20. Rezeptur nach Anspruch 1, wobei die funktionelle Beschichtung, die wahlweise bereitgestellt wird etwa 2 Gew.-% bis 20 Gew.-%, bevorzugt 2,5 Gew.-% bis 10 Gew.-%, auf den unbeschichteten Tablettenkern bezogen, darstellt.

21. Rezeptur nach Anspruch 20, wobei die funktionelle Beschichtung, die wahlweise über der äußeren festen kontinuierlichen Phase bereitgestellt wird, die Teilchen der inneren festen Phase darin eingebettet enthält, eine oder mehrere Filmbildner wie neutrales Methacrylsäureester-Polymer, Ethylcellulose, Celluloseacetat, Polyvinylalkohol-Maleinsäureanhydrid-Copolymere, Beta-Pinen-Polymere, Glycerylester von Holzharzen und dergleichen umfasst.

22. Rezeptur nach Anspruch 20, wobei geeignete Färbemittel der Beschichtung zugegeben werden.

23. Rezeptur nach Anspruch 1, wobei Weichmacher eingeschlossen sind, um die Eigenschaften und Charakteristiken von Polymeren, die in den Beschichtungen der inneren teilchenförmigen Phase und/oder auf der Beschichtung der komprimierten Tabletten verwendet werden, zu modifizieren.

24. Rezeptur nach Anspruch 23, wobei die Weichmacher unter Polymeren niederer Molmasse, Oligomeren, Copolymeren, Ölen, kleinen organischen Molekülen, Polyolen niedriger Molmasse mit aliphatischen Hydroxylen, Weichmachern vom Estertyp, Glykolethern, Poly(propylenglykol), Multiblockpolymeren, Einzelblockpolymeren, Poly(ethylenglykol) geringer Molmasse, Weichmachern vom Citratestertyp, Triacetin, Propylenglykol und Glycerin ausgewählt werden, wobei derartige Weichmacher auch Ethylenglykol, 1,2-Butylenglykol, 2,3-Butylenglykol, Styrolglykol, Diethylenglykol, Triethylenglykol, Tetraethylenglykol und andere Poly(ethylenglykol)verbindungen, Monopropylenglykolmonoisopropylether, Propylenglykolmonoethylether, Ethylenglykolmonoethylether, Diethylenglykolmonoethylether, Sorbitlactat, Ethyllactat, Butyllactat, Ethylglykolat, Dibutylsebazat, Actetyltributylcitrat, Triethylcitrat, Acetyltriethylcitrat, Tributylcitrat und Allylglykolat oder Kombinationen derselben umfassen können.

25. Rezeptur nach Anspruch 24, wobei die verwendeten Öle unter Erdnussöl, Sesamöl, Baumwollsamenöl, Maisöl und Olivenöl; Fettsäuren wie Ölsäure, Stearinsäure und Isostearinsäure; und Fettsäurestern wie Ethyloleat, Isopropylmyristat, Fettsäureglyceriden, Triglyeriden mittlerer Kette und acetylierten Fettsäureglyceriden ausgewählt werden.

26. Rezeptur nach Anspruch 1, wobei die Dosierform/Tablette einen Antiadhärenten, ein Gleitmittel, Schmiermittel, opak machendes Mittel, Färbemittel, Poliermittel, Säuerungsmittel, Alkalisiermittel, Antioxidationsmittel, Puffermittel und oberflächenaktives Mittel umfasst.

27. Rezeptur nach Anspruch 26, wobei der Antiadhärent unter Magnesiumstearat, Talk, Calciumstearat, Glycerylbehenat, Polyethylenglykolen, gehärtetem Pflanzenöl, Mineralöl, Stearinsäure und derartigen Materialien ausgewählt wird.

28. Rezeptur nach Anspruch 26, wobei das Gleitmittel unter Maisstärke, Talk, Calciumsilicat, Magnesiumsilicat, kolloidalem Siliciumdioxid, Siliciumhydrogel und dergleichen Materialen ausgewählt wird.

29. Rezeptur nach Anspruch 26, wobei das Schmiermittel unter Calciumstearat, Magnesiumstearat, Mineralöl, Stearinsäure und Zinkstearat und derartigen Materialien ausgewählt wird.

30. Rezeptur nach Anspruch 26, wobei das opak machende Mittel als solches oder in Kombination mit Färbemitteln wie Titandioxid und derartigen Materialien verwendet wird.

31. Rezeptur nach Anspruch 26, wobei das Färbemittel unter FD&C-Rot Nr. 3, FD&C-Rot Nr. 20, FD&C-Gelb Nr. 6, FD&C-Blau Nr. 2, D&C-Grün Nr. 5, D&C-Orange Nr. 5, D&C-Rot Nr. 8, Karamel und Eisen(III)oxid, Eisenrot, anderen FD&C-Farbstoffen und natürlichen Färbemitteln wie Traubenschalenextrakt, Rotebeetepulver, Beta-Karotin, Annato, Carmin, Curcumen, Paprika und derartigen Materialien ausgewählt wird.

32. Rezeptur nach Anspruch 26, wobei das Poliermittel unter Carnaubawachs, weißem Wachs und derartigen Materialien ausgewählt wird.

33. Rezeptur nach Anspruch 26, wobei das Säuerungsmittel unter Essigsäure, Aminosäure, Zitronensäure, Fumarsäure und anderen Alpha-Hydroxysäuren, wie Salzsäure, Ascorbinsäure und Salpetersäure und derartigen Materialien ausgewählt wird.

34. Rezeptur nach Anspruch 26, wobei das Alkalisiermittel unter Ammoniaklösung, Ammoniumcarbonat, Diethanolamin, Monoethanolamin, Kaliumhydroxid, Natriumborat, Natriumcarbonat, Natriumbicarbonat, Natriumhydroxid, Triethanolamin und Trolamin und derartigen Materialien ausgewählt wird.

35. Rezeptur nach Anspruch 26, wobei die Antioxidationsmittel unter Ascorbinsäure, Ascorbylpalmitat, butyliertem Hydroxyanisol, butyliertem Hydroxytoluol, Hypophosphonsäure, Monothioglycerin, Propylgallat, Natriumascorbat, Natriumbisulfit, Natriumformaldehydsulfoxylat und Natriummetabisulfit und derartigen Materialien ausgewählt werden.

36. Rezeptur nach Anspruch 26, wobei das Puffermittel unter Kaliummetaphosphat, Kaliumphosphat, einbasigem Natriumacetat und wasserfreiem Natriumcitrat und Natriumcitratdecahydrat und dergleichen Materialen ausgewählt wird.

37. Rezeptur nach Anspruch 1, wobei die Dosierform/Tablette oberflächenaktive Mittel umfasst, die das Benetzen des Tablettenkerns oder der Beschichtungsschichten verbessern.

38. Rezeptur nach Anspruch 37, wobei das oberflächenaktive Mittel Seife und synthetische Detergentien ist.

39. Rezeptur nach Anspruch 38, wobei die Seifen Fettsäurealkalimetall, Ammonium und Triethanolaminsalze umfassen.

40. Rezeptur nach Anspruch 38, wobei die Detergentien kationische Detergentien, beispielsweise Dimethyldialkylammoniumhalogenide, Alkylpyridiniumhalogenide und Alkylaminacetate; anionische Detergentien, beispielsweise Alkyl, Aryl und Olefinsulfonate, Alkyl, Olefin, Ether und Monoglyceridsulfate und Sulfosuccinate; nichtionische Detergentien, beispielsweise Fettaminoxide, Fettsäurealkanolamide und Poly(oxyethylen)-Blockpoly(oxypropylen)-Copolymere; und amphotere Detergentien, beispielsweise Alkylbetaaminopropionate und quartäre Ammoniumsalze von 2-Alkylimidazolin und Mischungen derselben sind.

41. Verfahren für das Herstellen einer osmo-mikroversiegelte Rezeptur verlängerter Freisetzung umfassend die folgenden Schritte:
i. Bilden einer osmo-mikroversiegelten inneren festen teilchenförmigen Phase durch Granulieren von Venlafaxin aktiv oder eines Salzes desselben mit einem oder mehreren Verdünnungsmitteln zum Erhöhen der Masse, Bindemittel zum Verleihen von Festigkeit/Härte dem einen oder den mehreren teilchenförmigen Osmogen(en) zum Bilden von osmotischem Druck durch die hydrophobe Beschichtung und das hydrophobe Polymer hindurch,
ii) Einbetten der inneren festen teilchenförmigen Phase in eine äußere feste kontinuierliche Phase, die ein oder mehrere hydrophile Polymere einschließt,
iii) Komprimieren der Zweiphasenmischung zu Tabletten,
iv) Beschichten der Tablette wahlweise mit einer funktionellen, Polymere enthaltenden Beschichtung.

42. Verfahren nach Anspruch 41, wobei die innere osmo-mikroversiegelte Teilchenbeschichtung durch Granulieren von Arzneimittel, Verdünnungsmittel, Bindemittel und Osmogenmischung erhalten wird, wobei die Dispersion des Beschichtungspolymers eine Matrix von Arzneimittel, Verdünnungsmittel, Osmogen und Polymer bildet. Nötigenfalls werden die Körnchen erneut granuliert, bis die gesamte Beschichtung aufgetragen ist.

43. Verfahren nach Anspruch 41, wobei die innere osmo-mikroversiegelte Teilchenbeschichtung durch Granulieren von Arzneimittel, Verdünnungsmittel und Bindemittel mit der Lösung von Osmogen erhalten wird. Die Granulierung wird noch weiter mit der Dispersion von hydrophobem Polymer fortgesetzt. Nötigenfalls werden die Körnchen erneut granuliert, bis die gesamte Beschichtung aufgetragen ist.

44. Verfahren nach Anspruch 41, wobei die innere osmo-mikroversiegelte Teilchenbeschichtung durch Granulieren von Arzneimittel, Verdünnungsmittel und Osmogen mit der Lösung von Bindemittel erhalten wird. Die Granulierung wird noch weiter mit der Dispersion von hydrophobem Polymer fortgesetzt. Nötigenfalls werden die Körnchen erneut granuliert, bis die gesamte Beschichtung aufgetragen ist.

45. Verfahren nach Anspruch 41, wobei die innere osmo-mikroversiegelte Teilchenbeschichtung durch teilweises Granulieren von Arzneimittel, Verdünnungsmittel und Osmogenmischung mit der Dispersion von Beschichtungspolymer, die die Matrix von Arzneimittel, Verdünnungsmittel, Osmogen und Polymer bildet, erhalten wird. Die Körnchen werden noch weiter auf einem Wirbelbettprozessor mit der verbleibenden Menge des hydrophoben Polymers beschichtet.

46. Verfahren nach Anspruch 41, wobei die inneren osmo-mikroversiegelten Teilchen durch Granulieren des Arzneimittels, Osmogens und Bindemittels erhalten werden. Die Körnchen werden noch weiter auf einem Wirbelbettprozessor mit dem hydrophoben Beschichtungspolymer beschichtet.

47. Verfahren nach Anspruch 41, wobei die inneren osmo-mikroversiegelten Teilchen durch Granulieren des Arzneimittels, Bindemittels und Verdünnungsmittels unter Anwendung einer Lösung von Osmogen erhalten werden. Die Körnchen werden noch weiter auf einem Wirbelbettprozessor mit dem hydrophoben Beschichtungspolymer beschichtet.

48. Verfahren nach Anspruch 41, wobei die inneren osmo-mikroversiegelten Teilchen durch Extrusionsspäronisierung einer nassgemischten Masse von Arzneimittel, Bindemittel, Verdünnungsmittel und Osmogen erhalten werden. Die erhaltenen Minisphären werden noch weiter auf einem Wirbelbettprozessor mit dem hydrophoben Beschichtungspolymer beschichtet.

## Revendications

1. Préparation osmo-microscellée à libération prolongée comprenant une phase particulaire osmo-microscellée solide interne consistant en une quantité thérapeutiquement efficace de venlafaxine, active ou sel de cette dernière, et en au moins un osmogène/agent ou osmopolymère, un diluant, un liant et une membrane polymérique hydrophobe formant le noyau ; une phase continue solide externe consistant en un polymère hydrophile hydrosoluble et/ou gonflant, tassée dans des comprimés et recouverte, en option, d'un enrobage fonctionnel.

2. Préparation selon la revendication 1, dans laquelle le rapport phase particulaire osmo-microscellée interne/phase continue externe se situe dans les limites de 0,3 : 1 à 10 :1, préférablement de 0,5 :1 à environ 4 :1.

3. Préparation selon la revendication 1, dans laquelle la phase particulaire solide interne contient un médicament actif ou un sel dudit médicament en une quantité située entre les limites d'environ 5 % à 75 %, préférablement d'environ 7 % à 65 % en poids, de l'éthylcellulose et/ou du cellulose acétate en une quantité située entre les limites de 0,5 % à 65 % en poids, préférablement de 2 % à 45 % en poids, du chlorure de sodium et/ou du mannitol dans les limites de 0,01 % à 25 % en poids, préférablement de 0,05 % à 10 % en poids, du polyvinyle pyrolidone et/ou de l'hydroxypropylméthylcellulose (faible viscosité) dans les limites de 0,1 % à 10 % en poids, préférablement de 0,5 % à 8 % en poids, et elle peut contenir de la cellulose microcristalline et/ou du lactose en une quantité située entre les limites d'environ 0 % à 90 % en poids, préférablement de 20 % à 80 % en poids, les pourcentages ci-dessus ayant pour base le poids de la phase particulaire solide interne.

4. Préparation selon la revendication 1, dans laquelle la phase particulaire solide interne contient le polymère hydrophobe en une quantité située dans les limites d'environ 0,5 % à 65 % en poids, préférablement d'environ 2 % à 45 % en poids de la phase particulaire solide interne.

5. Préparation selon la revendication 4, dans laquelle le polymère hydrophobe est utilisé sous la forme d'une solution non aqueuse, d'une suspension aqueuse, d'une émulsion aqueuse ou d'une solution solvantée organique contenant de l'eau.

6. Préparation selon la revendication 4, dans laquelle le polymère hydrophobe est sélectionné parmi l'éthylcellulose, la méthylcellulose, le copolymère amino-méthacrylate, des copolymères de l'acide méthacrylique, le copolymère acide méthacrylique/éthyl ester d'acide acrylique, le copolymère neutre d'esters de l'acide méthacrylique, le copolymère diméthylaminoéthyle méthacrylate/esters de l'acide méthacrylique, l'acétate de cellulose, les copolymères vinyl-méthyl-éther/anhydride maléique.

7. Préparation selon la revendication 1, dans laquelle la phase particulaire solide contient un osmogène en une quantité située dans les limites d'environ 0,01 % à environ 25 % en poids, préférablement de 0,05 % à environ 10 % en poids de la phase particulaire solide interne.

8. Préparation selon la revendication 7, dans laquelle les osmogènes incluent des composés organiques et inorganiques tels que des sels, des acides, des bases, des agents chélateurs, du chlorure de sodium, du chlorure de lithium, du chlorure de magnésium, du sulfate de magnésium, du sulfate de lithium, du chlorure de potassium, du sulfite de sodium, du bicarbonate de calcium, du sulfate de sodium, du sulfate de calcium, du lactate de calcium, du d-mannitol, de l'urée, de l'acide tartrique, du raffinose, du saccharose, du monohydrate d' alpha-d-lactose, du glucose, du sorbitol et les autres matières similaires ou équivalentes et l'association de ceux-ci.

9. Préparation selon la revendication 1, dans laquelle la phase particulaire solide contient un liant en une quantité située dans les limites d'environ 0,1 % à environ 10 % en poids, préférablement de 0,5 % à environ 8 % en poids de la phase particulaire solide interne.

10. Préparation selon la revendication 9, dans laquelle le liant est sélectionné parmi le polyacrylamide, le poly-N-vinylamide, le poly-N-vinyl-acétamide, le polyvinyle pyrolidone, l'amidon, le lactose, l'amidon de maïs modifié, des sucres, la gomme d'acacia, l'acide alginique, la carboxyméthylcellulose sodique, le gacanthe, la gélatine, le glucose liquide, la méthylcellulose, l'amidon prégélatinisée, le polyéthylèneglycol, la gomme de guar, le polysaccharide, les bentonites, les sucres invertis, le collagène, l'albumine, le polypropylèneglycol, le copolymère polyoxyéthylène-polypropylène, le polyéthylène-ester, le polyéthylène-sorbitan-ester, l'oxyde de polyéthylène et l'hydroxypropylméthylcellulose et les autres matières similaires ou équivalentes ou une association de ceux-ci.

11. Préparation selon la revendication 10, dans laquelle la viscosité de l'hydroxypropyl-méthylcellulose est une faible viscosité, préférablement inférieure à 0,01 Pa.s (10 cps) et plus préférablement de 0,002 à 0,005 Pa.s (2 à 5 cps).

12. Préparation selon la revendication 1, dans laquelle la phase particulaire solide interne contient un diluant en une quantité située dans les limites de 0 % à 90 % en poids ou préférablement d'environ 20 % à 80 % en poids de la phase particulaire solide interne.

13. Préparation selon la revendication 12, dans laquelle le diluant est une substance inerte utilisée en tant qu'excipients pour créer les propriétés d'écoulement en vrac et la compression désirées, caractéristique requise dans la préparation des comprimés.

14. Préparation selon la revendication 12, dans laquelle le diluant est sélectionné parmi le phosphate de calcium dibasique, le kaolin, le lactose, le saccharose, le mannitol, la cellulose microcristalline, la cellulose en poudre, le carbonate de calcium précipité, le sorbitol et l'amidon et les matières semblables.

15. Préparation selon la revendication 1, dans laquelle la phase particulaire solide interne a une dimension particulaire moyenne située entre les limites d'environ 0,01 micromètre à environ 2 mm, préférablement de 50 micromètres à environ 0,5 mm.

16. Préparation selon la revendication 1, dans laquelle ladite phase continue solide externe contient des polymères hydrophiles en une quantité située dans les limites d'environ 3 % à 60 % en poids, préférablement d'environ 10 % à 55 % en poids de la forme pharmaceutique/du comprimé non enrobé(e).

17. Préparation selon la revendication 16, dans laquelle le polymère hydrophile est sélectionné parmi l'hydroxyéthylcellulose, l'hydroxypropylcellulose, l'alginate de sodium, le carbomère (Carbopol^{™}), la carboxyméthylcellulose sodique, la gomme de xanthane, la gomme de guar, la gomme de caroube, l'acétate de polyvinyle, l'alcool de polyvinyle et l'hydroxypropylméthylcellulose.

18. Préparation selon la revendication 16, dans laquelle ladite phase continue solide externe inclut un(e) ou plusieurs charges ou excipients en une quantité située dans les limites d'environ 1 % à 70 % en poids, plus préférablement de 10 % à 40 % en poids de la forme pharmaceutique/du comprimé non enrobé(e).

19. Préparation selon la revendication 16, dans laquelle ladite phase continue solide externe inclut le taux recommandé de régulateurs de glissement, de lubrifiants, de liants déshydratés et d'anti-adhérants.

20. Préparation selon la revendication 1, dans laquelle l'enrobage fonctionnel fourni en option est l'équivalent d'environ 2 % à 20 % en poids, préférablement de 2,5 % à 10 % en poids du noyau du comprimé non enrobé.

21. Préparation selon la revendication 20, dans laquelle la couche d'enrobage fonctionnel fourni en option recouvrant la phase continue solide externe renfermant les particules de la phase solide interne, inclut un ou plusieurs agents filmogènes tels que le polymère neutre d'esters de l'acide méthacrylique, l'éthylcellulose, l'acétate de cellulose, les copolymères alcool polyvinylique-anhydride maléique, les polymères bêta-pinène, les esters du glycéryle et des résines de bois et autres agents filmogènes semblables.

22. Préparation selon la revendication 20, dans laquelle des agents colorants adéquats sont ajouté à l'enrobage.

23. Préparation selon la revendication 1, dans laquelle des plastifiants sont inclus pour modifier les propriétés et la caractéristique des polymères utilisés dans les enrobages de la phase particulaire interne et/ou sur l'enrobage des comprimés.

24. Préparation selon la revendication 23, dans laquelle les plastifiants sont sélectionnés parmi des polymères à faible poids moléculaire, des polymères à faible poids moléculaire, des oligomères, des copolymères, des huiles, de petites molécules organiques, des polyols de faible poids moléculaire ayant des hydroxyles aliphatiques, des plastifiants de type ester, des glycoléthers, du poly(propylène) glycol, des polymères multi-blocs, des polymères monoblocs, du poly(éthylène) glycol de faible poids moléculaire, des plastifiants de type ester de citrate, de la triacétine, du propylène glycol et de la glycérine, dans laquelle de tels plastifiants peuvent aussi inclure de l'éthylène glycol, du 1,2-butylène glycol, du 2,3-butylène glycol, du styrène glycol, du diéthylène glycol, du triéthylène glycol, du tétraéthylène glycol et d'autres composés à base de poly(éthylène) glycol, de l'éther monoisopropylique de monopropylèneglycol, de l'éther monoéthylique de propylèneglycol, de l'éther monoéthylique d'éthylène glycol, de l'éther monoéthylique de diéthylène glycol, du lactate de sorbitol, du lactate d'éthyle, du lactate de butyle, du glycolate d'éthyle, du dibutylsébacate, de l'acétyltributylcitrate, du citrate triéthylique, de l'acétyltriéthylcitrate, du tributylcitrate et du glycolate d'allyle ou une association de ceux-ci.

25. Préparation selon la revendication 24, dans laquelle les huiles utilisées sont sélectionnées parmi l'huile d'arachide, l'huile de sésame, l'huile de coton, l'huile de maïs et l'huile d' olive ; les acides gras, tels que l'acide oléique, l'acide stéarique et l'acide isostéarique ; et les esters des acides gras tels que l'oléate éthylique, le myristate isopropylique, les glycérides des acides gras, les triglycérides à chaîne moyenne et les glycérides des acides gras acétylés.

26. Préparation selon la revendication 1, dans laquelle la forme pharmaceutique/le comprimé comprend un anti-adhérant, un régulateur de glissement, un lubrifiant, un opacifiant, un colorant, des agents de polissage, un acidifiant, un alcalinisant, un antioxydant, un tampon et un agent tensioactif.

27. Préparation selon la revendication 26, dans laquelle l' anti-adhérant est sélectionné parmi le stéarate de magnésium, le talc, le stéarate de calcium, le béhénate de glycéryle, les polyéthylèneglycols, l'huile végétale hydrogénée, l'huile minérale, l'acide stéarique et les matières semblables.

28. Préparation selon la revendication 26, dans laquelle le régulateur de glissement est sélectionné parmi l'amidon de maïs, le talc, le silicate de calcium, le silicate de magnésium, le dioxyde de silicium colloïdal, l'hydrogel de silicium et les matières semblables.

29. Préparation selon la revendication 26, dans laquelle le lubrifiant est sélectionné parmi le stéarate de calcium, le stéarate de magnésium, l'huile minérale, l'acide stéarique et le stéarate de zinc et les matières semblables.

30. Préparation selon la revendication 26, dans laquelle l'opacifiant est utilisé seul ou en association avec un colorant tel que le dioxyde de titane et les matières semblables.

31. Préparation selon la revendication 26, dans laquelle le colorant est sélectionné parmi le FD&C Rouge No. 3, le FD&C Rouge No. 20, le FD&C Jaune No. 6, le FD&C Bleu No. 2, le D&C Vert No. 5, le D&C Orange No. 5, le D&C Rouge No. 8, le caramel, et l'oxyde ferrique, le rouge, d'autres colorants FD&C et des colorants naturels tels que l'extrait de peau de raisin, la poudre de betterave rouge, le bêta-carotène, le rocouyer, le carmin, le curcuma, le paprika et les matières semblables.

32. Préparation selon la revendication 26, dans laquelle l'agent polissant est sélectionné parmi la cire de carnauba, la cire blanche et les matières semblables.

33. Préparation selon la revendication 26, dans laquelle l'acidifiant est sélectionné parmi l'acide acétique, l'aminoacide, l'acide citrique, l'acide fumarique et d'autres alphahydroxy acides, tels que l'acide chlorhydrique, l'acide ascorbique et l'acide nitrique et les matières semblables.

34. Préparation selon la revendication 26, dans laquelle l'alcalinisant est sélectionné parmi l'ammoniaque, le carbonate d'ammonium, la diéthanolamine, la monoéthanolamine, l'hydroxyde de potassium, le borate de sodium, le carbonate de sodium, le bicarbonate de sodium, l'hydroxyde de sodium, la triéthanolamine, et la trolamine et les matières semblables.

35. Préparation selon la revendication 26, dans laquelle les antioxydants sont sélectionnés parmi l'acide ascorbique, le palmitate ascorbylique, l'hydroxyamisole butylé, l'hydroxytoluène butylé, l'acide hypophosphoreux, le monothioglycérol, le propylgallate, l'ascorbate de sodium, le bisulfite de sodium, le sulfoxylate de formaldehyde sodique et le métabisulfite de sodium, et les matières semblables.

36. Préparation selon la revendication 26, dans laquelle le tampon est sélectionné parmi le métaphosphate de potassium, le phosphate de potassium, l'acétate de sodium monobasique et le citrate de sodium anhydre et déhydrat, et les matières semblables.

37. Préparation selon la revendication 1, dans laquelle la forme pharmaceutique/le comprimé inclut un agent tensioactif qui améliore la mouillance du noyau du comprimé ou des couches d'enrobage.

38. Préparation selon la revendication 37, dans laquelle l'agent tensioactif est un savon et un détergent synthétique.

39. Préparation selon la revendication 38, dans laquelle les savons incluent le métal alcalin d'acides gras, l'ammonium et des sels de triéthanolamine.

40. Préparation selon la revendication 38, dans laquelle les détergents sont des détergents cationiques, par exemple, des halogénures de diméthyl-dialkyl-ammonium, des halogénures d'alkyl-pyridinium, et des acétates d'alkylamine ; des détergents anioniques, par exemple des sulfonates d'alkyle, d'aryle et d'oléfine, des sulfates d'alkyle, d'oléfine, d'éther et de monoglycérides, et des sulfosuccinates ; des détergents nonioniques, par exemple des oxydes d'amines gras, des alkanolamides d'acides gras, et des copolymères poly(oxyéthylène)-poly(oxypropylène) en blocs, et des détergents amphotériques, par exemple des alkyl-bêta-aminopropionates et des sels d'ammonium quaternaires 2-alkylimidazoline ; et des mélanges de ceux-ci.

41. Processus pour préparer une préparation osmo-microscellée à libération prolongée, comprenant les étapes suivantes :
i. formation d'une phase particulaire solide interne par granulation de la venlafaxine, active ou d'un de ses sels, avec un ou plusieurs diluants pour augmenter la masse, un liant pour conférer robustesse et dureté aux particules, un ou plusieurs osmogènes pour générer une pression osmotique dans l'enrobage hydrophobe et le polymère hydrophobe.
ii. le tassement de la phase particulaire solide interne dans une phase continue solide externe incluant un ou plusieurs polymère(s) hydrophile(s).
iii. la compression du mélange biphasique pour obtenir un comprimé.
iv. l'enrobage facultatif du comprimé avec un enrobage fonctionnel contenant des polymères.

42. Processus selon la revendication 41, dans lequel l'enrobage particulaire osmo-microscellé interne est obtenu par granulation du médicament, du diluant, du liant et du mélange osmogène, la dispersion du polymère d'enrobage formant une matrice de médicament, diluant, osmogène et le polymère. Si requis, les granules peuvent être regranulés jusqu'à ce que tout l'enrobage soit appliqué.

43. Processus selon la revendication 41, dans lequel l'enrobage particulaire osmo-microscellé interne est obtenu par granulation du médicament, du diluant et du liant avec la solution d'osmogène. La granulation se poursuit, en outre, avec la dispersion du polymère hydrophobe. Si requis, les granules sont regranulés jusqu'à ce que tout l'enrobage soit appliqué.

44. Processus selon la revendication 41, dans lequel l'enrobage particulaire osmo-microscellé interne est obtenu par granulation du médicament, du diluant et de l' osmogène avec la solution de liant. La granulation se poursuit, en outre, avec la dispersion du polymère hydrophobe. Si requis, les granules sont regranulés jusqu'à ce que tout l'enrobage soit appliqué.

45. Processus selon la revendication 41, dans lequel l'enrobage particulaire osmo-microscellé interne est obtenu par granulation partielle du médicament, du diluant et du mélange osmogène, la dispersion du polymère d'enrobage formant une matrice de médicament, de diluant, d' osmogène et le polymère. Les granules sont, de plus, enrobés dans un processeur à lit fluidisé avec le polymère d'enrobage hydrophobe.

46. Processus selon la revendication 41, dans lequel l'enrobage particulaire osmo-microscellé interne est obtenu par granulation du médicament, de l'osmogène et du liant. Les granulés sont, en outre, enrobés dans un processeur à lit fluidisé avec le polymère d'enrobage hydrophobe.

47. Processus selon la revendication 41, dans lequel l'enrobage particulaire osmo-microscellé interne est obtenu par granulation du médicament, du liant et du diluant en utilisant une solution d'osmogène. Les granulés sont, en outre, enrobés dans un processeur à lit fluidisé avec le polymère d'enrobage hydrophobe.

48. Processus selon la revendication 41, dans lequel l'enrobage particulaire osmo-microscellé interne est obtenu par extrusion-sphéronisation d'une masse mélangée humide de médicament, de liant, de diluant et d'osmogène. Les mini-sphérules obtenues sont, en outre, enrobées dans un processeur à lit fluidisé avec le polymère d'enrobage hydrophobe.
